(19) **Europäisches Patentamt**
**European Patent Office**
**Office européen des brevets**

(11) **EP 1 599 836 B1**

(12) **EUROPEAN PATENT SPECIFICATION**

(45) Date of publication and mention
of the grant of the patent:
**21.04.2010 Bulletin 2010/16**

(51) Int Cl.:
***G06T 11/00*** (2006.01)

(21) Application number: **04709286.1**

(86) International application number:
**PCT/IB2004/000386**

(22) Date of filing: **09.02.2004**

(87) International publication number:
**WO 2004/072905 (26.08.2004 Gazette 2004/35)**

(54) **SYSTEM AND METHOD FOR HELICAL CONE-BEAM COMPUTED TOMOGRAPHY WITH EXACT RECONSTRUCTION**

SYSTEM UND VERFAHREN FÜR SPIRALFÖRMIGE 3D-COMPUTERTOMOGRAPHIE MIT EXAKTER REKONSTRUKTION

SYSTEME ET PROCEDE DESTINES A LA TOMOGRAPHIE PAR ORDINATEUR A FAISCEAU CONIQUE HELICOIDAL AVEC RECONSTRUCTION EXACTE

(84) Designated Contracting States:
**AT BE BG CH CY CZ DE DK EE ES FI FR GB GR HU IE IT LI LU MC NL PT RO SE SI SK TR**

(30) Priority: **14.02.2003 US 447426 P**
**25.06.2003 US 482380 P**

(43) Date of publication of application:
**30.11.2005 Bulletin 2005/48**

(73) Proprietor: **Koninklijke Philips Electronics N.V.**
**5621 BA Eindhoven (NL)**

(72) Inventors:
• **HEUSCHER, Dominic, J.**
**Cleveland, OH 44143 (US)**
• **NOO, Frederic, N.**
**Cleveland, OH 44143 (US)**
• **BROWN, Kevin, M.**
**Cleveland, OH 44143 (US)**
• **PACK, Jed, D.**
**Cleveland, OH 44143 (US)**

(74) Representative: **Damen, Daniel Martijn et al**
**Philips**
**Intellectual Property & Standards**
**P.O. Box 220**
**5600 AE Eindhoven (NL)**

(56) References cited:
**WO-A-03/015634    US-A- 6 130 930**

• **NOO F ET AL: "Exact helical reconstruction using native cone-beam geometries" PHYSICS IN MEDICINE AND BIOLOGY, 7 DEC. 2003, IOP PUBLISHING, UK, vol. 48, no. 23, 7 December 2003 (2003-12-07), pages 3787-3818, XP002279259 ISSN: 0031-9155**
• **KATSEVICH A ET AL: "Evaluation and empirical analysis of an exact FBP algorithm for spiral cone-beam CT" MEDICAL IMAGING 2003: IMAGE PROCESSING;SAN DIEGO, CA, UNITED STATES FEB 17-20 2003, vol. 5032 II, 2003, pages 663-674, XP002279112 Proc SPIE Int Soc Opt Eng; Proceedings of SPIE - The International Society for Optical Engineering 2003 cited in the application**

**Description**

The following relates to the diagnostic imaging arts. It finds particular application in helical conebeam computed tomography imaging, and will be described with particular reference thereto. However, it also finds application in other types of tomographic imaging.

Single-slice x-ray transmission computed tomography imaging systems employ limited beam dimensions, such as an essentially planar fan-beam that is rotated in a circular orbit around an imaging subject. The acquired projection data is reconstructed to generate an image slice. In multi-slice fan-beam imaging, data are acquired for a plurality of axially spaced-apart circular orbits, and reconstructed to produce a corresponding plurality of axially spaced-apart image slices. Multi-slice computed tomography imaging is conducive to fast and accurate image reconstruction due to the simplified fan-beam geometry. In one suitable reconstruction method, each slice is reconstructed separately using convolution backprojection. In another reconstruction method, the fan-beam projection data are rebinned into substantially parallel-ray views that are backprojected to reconstruct the image. Even in multi-slice methods, the rate of data acquisition is limited due to the small volume sampled by the essentially planar fan-beam and the discontinuous axially stepped orbiting of the x-ray source.

Helical conebeam computed tomography was developed to overcome these data acquisition rate constraints. A continuous helical x-ray source orbit is combined with a conebeam geometry that interacts with a three-dimensional volume to enable much higher data acquisition rates. A two-dimensional array of x-ray detector elements is arranged to receive the conebeam after it passes through an examination region, in which the imaging subject is disposed. The detector array can be flat (flat detector geometry), curved (curved detector geometry), or otherwise shaped.

A disadvantage of helical conebeam computed tomography is that reconstruction of the conebeam projection data into an image representation is computationally complex. A given voxel of the image volume is sampled by non-coplanar projections spanning a portion of the helical trajectory of the x-ray source. In contrast, with the fan-beam geometry projections for a given voxel are substantially coplanar, that is, lie in a single plane.

Various conebeam reconstruction methods have been developed. Some of these techniques, such as the N-PI method, are not exact, in that they fail to accurately account for the cone angle. These inexact reconstruction methods introduce artifacts into the reconstructed image because they do not accurately account for the cone angles of the projections, that is, angular deviations of the projections from the axial plane. By employing various approximations relating to the cone angle, inexact reconstruction methods can provide rapid reconstruction with acceptably limited image artifacts for data acquired using small cone angles. However, these inexact reconstructed images become increasingly degraded by artifacts as the cone angle increases. Conebeams with large cone angles provide large beam interaction volumes and advantageously enable fast data acquisition.

For data acquired with large cone angles, an exact reconstruction method is preferably used that accurately accounts for projection components in the cone angle direction. Prior exact reconstruction methods are more computationally intense than inexact methods due to the more complex conebeam geometry. An exact conebeam reconstruction method has been developed by Katsevich (see for example Katsevich et al, Proceedings SPIE Medical Imaging Conference, San Diego, California (February 2003), pp.663-674).

However, this exact conebeam reconstruction method employs a voxel-based coordinate system that does not comport with conebeam data acquisition detector geometries. Conversion of acquired projection data, which are defined using coordinates such as x-ray detector row and column values, projection angle coordinates, helix angle, or the like, into a detector-independent voxel-based coordinate system is computationally challenging and involves additional rebinning operations that substantially increase reconstruction time. Moreover, the previous exact conebeam reconstruction method is not readily adapted to existing backprojector processing pipelines such as are commonly employed in reconstructing parallel-rebinned projection data.

The present invention contemplates an improved apparatus and method that overcomes the aforementioned limitations and others.

According to one aspect, a conebeam computed tomography imaging system is disclosed. An x-ray source produces an x-ray conebeam directed into an examination region. The x-ray source is arranged to traverse a generally helical trajectory around the examination region. An x-ray detector array is arranged to detect the x-ray conebeam after passing through the examination region. The x-ray detector array generates projection data in native scan coordinates defined with reference to the detector array. An exact reconstruction processor performs an exact reconstructing of conebeam projection data produced by the detector array into an image representation. The reconstructing is performed in the native scan coordinates. The reconstruction processor includes a derivative processor that computes a derivative of the projection data with respect to a helix angle of the helical trajectory at fixed projection direction to generate differentiated projection data. The reconstruction also further includes a convolution processor that convolves the differentiated projection data with a kernel function to produce filtered projection data, the convolving being performed in the native scan coordinates and a backprojector that backprojects the filtered projection data to obtain an image representation.

According to another aspect, a conebeam computed tomography imaging system is disclosed that produces conebeam computed tomography projection data having native scan coordinates that include at least a helix angle, a projection fan coordinate, and a projection cone coordinate. The imaging system includes a means for performing an exact reconstructing of the conebeam projection data into an image representation. The reconstructing is performed in the native scan coordinates. The means for reconstructing includes a means for computing filtered projection data. The means for computing includes a differentiating means for computing a derivative of projection data with respect to the helix angle at fixed projection direction and a convolving means for convolving projection data with a kernel function. The convolving is performed in the native scan coordinates. A means is provided for backprojecting the filtered projection data to obtain an image representation.

According to another yet aspect, an exact reconstruction method is provided for reconstructing conebeam computed tomography projection data having native scan coordinates that include at least a helix angle, a projection fan coordinate, and a projection cone coordinate. The method is performed in the native scan coordinates. Filtered projection data is computed by a combination of computing a derivative of projection data with respect to the helix angle at fixed projection direction and convolving projection data with a kernel function. The convolving is performed in the native scan coordinates. The filtered projection data is backprojected to obtain an image representation.

One advantage resides in fast, exact image reconstruction of conebeam imaging data acquired using a large cone angle.

Another advantage resides in simplified image reconstruction computations by obviating rebinning operations associated with transforming tomographic projection data into a detector-independent voxel-based coordinate system.

Another advantage resides in optional incorporation of a backprojector pipeline configured for processing parallel-rebinned projection data in performing exact reconstruction of conebeam projection data.

Yet another advantage resides in straightforward accommodation of various detector geometries such as flat detector geometries and curved detector x-ray source-focused detector geometries.

Numerous additional advantages and benefits will become apparent to those of ordinary skill in the art upon reading the following detailed description of the preferred embodiments.

The invention may take form in various components and arrangements of components, and in various process operations and arrangements of process operations. The drawings are only for the purpose of illustrating preferred embodiments and are not to be construed as limiting the invention.

FIGURE 1 diagrammatically shows a helical conebeam computed tomography imaging system including exact reconstruction of acquired conebeam projection data.

FIGURE 2 diagrammatically shows a detector-based coordinate system for a source-focused curved detector geometry.

FIGURE 3 diagrammatically shows a detector-based coordinate system for a flat detector geometry.

FIGURE 4 diagrammatically shows geometric aspects related to $\pi$-lines and $\kappa$-planes.

FIGURE 5 diagrammatically shows the $\cos\beta$ cone angle length normalization for projection data in a source-focused curved detector geometry.

FIGURE 6 shows exemplary $\kappa$-curves for a source-focused curved detector panel with sixteen detector rows, with a Tam-Danielsson window indicated by shading.

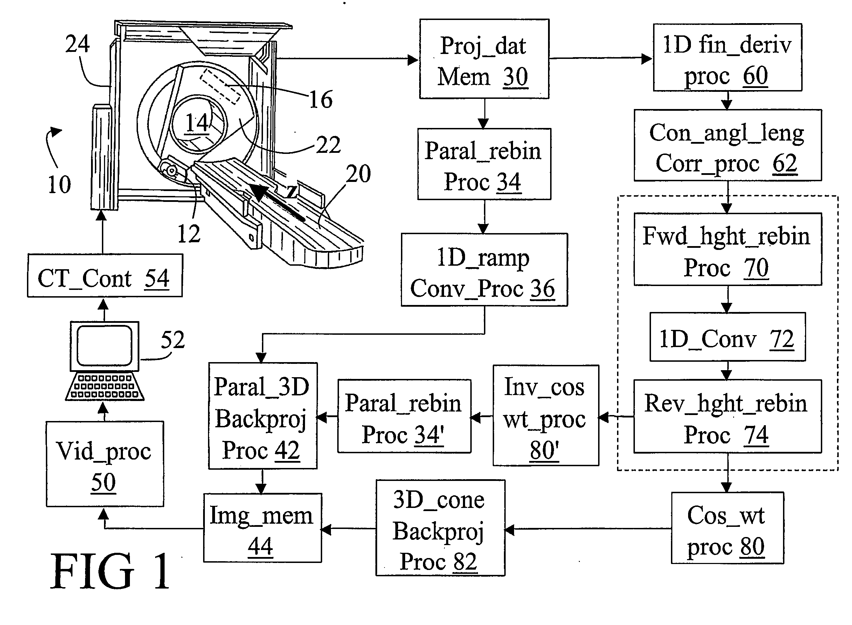

With reference to FIGURE 1, a helical conebeam computed tomography imaging scanner **10** includes an x-ray source **12** that projects an x-ray conebeam into an examination region **14**. After passing through the examination region, the x-ray conebeam is detected by a two-dimensional x-ray detector **16** (shown diagrammatically in phantom in FIGURE 1) that includes an array of detector elements arranged to detect the x-ray conebeam after passing through the examination region **14**.

To effect a helical trajectory of the x-ray source **12** about an imaging subject, the imaging subject is placed on a couch **20** or other support. The couch moves linearly along a z-direction as indicated. The x-ray source **12** and the x-ray detector **16** are oppositely mounted respective to the examination region **14** on a rotating gantry **22,** such that rotation of the gantry **22** effects rotation of the x-ray source **12**. Rotation of the gantry **22** along with simultaneous, continuous linear motion of the couch **20** effects a helical trajectory of the x-ray source **12** around the imaging subject disposed on the couch **20.**

The x-ray detector **16** is shown mounted on the rotating gantry **22** such that it rotates along with the x-ray source **12** to intercept the x-ray conebeam throughout the helical trajectory. However, it is also contemplated to replace the x-ray detector **16** by an x-ray detector band mounted around a stationary gantry **24**.

In operation, during helical orbiting of the x-ray source **12** relative to the imaging subject, the x-ray conebeam is projected into the examination region **14** where it interacts with the imaging subject. Some portion of the x-rays are absorbed by the imaging subject to produce a generally spatially varying attenuation of the x-ray conebeam. The x-ray detector **16** measures the x-ray intensities across the conebeam to generate x-ray absorption data that is stored in a projection data memory **30**.

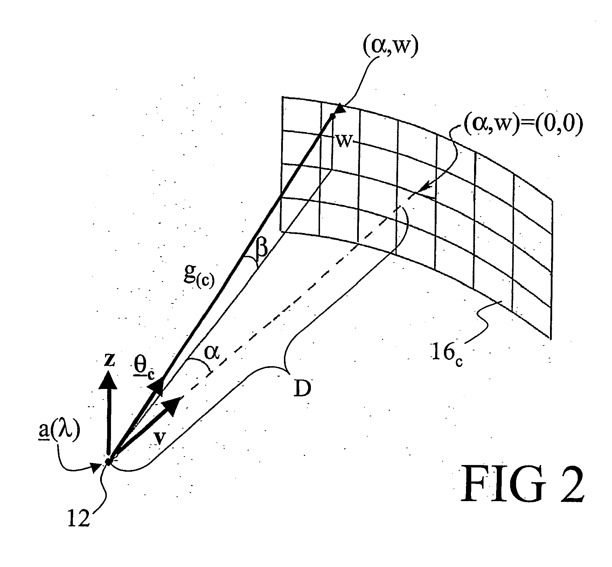

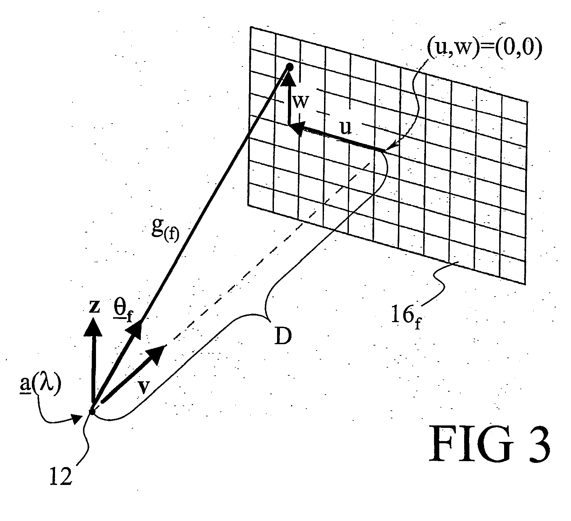

With continuing reference to FIGURE 1 and with further reference to FIGURES 2 and 3, the detector **16** is preferably one of a source-focused curved detector **16$_c$** shown in FIGURE 2 and a flat detector **16$_f$** shown in FIGURE 3. The detector-based coordinate system of the projection data stored in the projection data memory **30** depends upon the detector geometry, such as the curved geometry of the source-focused curved detector **16$_c$** shown in FIGURE 2, or the flat detector geometry of the flat detector **16$_f$** of FIGURE 3. In both geometries, a detector-based coordinate v is directed from the x-ray source **12** toward a center of the x-ray detector **16**. Native scan coordinates for the source-focused curved detector geometry and for the flat detector geometry are given in Table I.

**Table I - Native Scan Coordinates Systems**

| Parameter | Stationary coord. | Flat detector coord. | Curved detector coord. |
|---|---|---|---|
| In-slice | x | u | $\alpha$ |
| In-slice | y | v | v |
| Axial | z | w | w, $\beta$ |
| Helix angle | --- | $\lambda$ | $\lambda$ |

The (x,y,z) stationary coordinate system is a conventional Cartesian coordinate system and is provided for reference. The helix angle $\lambda$ identifies an angular position of the x-ray source **12** along the helical orbit.

With particular reference to FIGURE 2, in the source-focused curved detector geometry a projection fan coordinate $\alpha$ indicates the projection angle in the fan angle direction, while the a projection cone angle coordinate $\beta$ indicates the projection angle in the cone angle direction. A projection $g_{(c)}$ lies along a projection direction vector $\theta c$ in the curved detector geometry, and has coordinates $g_{(c)}(\lambda, \alpha, w)$ where w=D tan($\beta$) and D is a source-to-detector distance from the x-ray source **12** to a center of the detector **16$_c$**. The center of the curved detector **16$_c$** is given by coordinates ($\lambda$, 0, 0), that is, $\alpha$=w=0. The coordinate w is parallel to the axial or z-direction. The detector **16$_c$** is not curved along the axial or z-direction, but is curved along the fan direction, that is, along the direction corresponding to the angle coordinate $\alpha$. The detector curvature along the angle coordinate $\alpha$ is selected so that all detector elements for a given angle coordinate $\beta$ are substantially equidistant from the x-ray source **12**. That is, the detector curvature along the angle coordinate $\alpha$ is source-focused.

A vertex position $\underline{a}(\lambda)$ of the x-ray source **12** along the helical trajectory is given by helix angle $\lambda$ for the curved detector geometry. In the stationary (x, y, z) coordinate system, the vertex points $\underline{a}(\lambda)$ of the helix, that is, the trajectory of the x-ray source **12,** is given by:

$$\underline{a}(\lambda) = \left[ R_o \, \cos(\lambda + \lambda_o), R_o \, \sin(\lambda + \lambda_o), z_o + P\frac{\lambda}{2\pi} \right] . \qquad (1)$$

where $R_o$ is a source distance, that is, a distance from the x-ray source **12** to the helix axis, P is the helical pitch, that is, the axial or z-distance the x-ray source moves relative to the imaging subject over a single helical trajectory turn, and $\lambda_o$ and $z_o$ are a reference helix angle and reference axial position, respectively.

With particular reference to FIGURE 3, in the flat detector coordinate system the coordinate u rotates with respect to the stationary x-coordinate during helical orbiting of the x-ray source. A projection $g_{(f)}$ lies along a projection direction vector $\underline{\theta}_f$ in the flat detector geometry, and has coordinates $g_{(f)}(\lambda, u, w)$. (As noted previously, the symbol $\lambda$ is used herein for the helix angle of the flat detector geometry, rather than the more conventional helix angle symbol $\theta$). This projection is taken along a line from the x-ray source **12** toward detector coordinate (u, w) of the flat detector **16$_f$.** The center of the flat detector **16$_f$** is given by coordinates ($\lambda$, 0, 0), that is, u=v=0. The coordinate w is parallel to the axial or z-direction. The source-to-detector distance D of the curved geometry is also defined for the flat detector geometry. Equation (1) specifying the vertex position $\underline{a}(\lambda)$ of the x-ray source **12** in the stationary (x,y,z) coordinate system also applies to the flat detector geometry of FIGURE 3.

The detector arrays **16$_c$**, **16$_f$** are shown for convenience as having a small number of detector elements. In FIGURE 2, the array of detector elements of the curved detector **16$_c$** is represented by grid lines that indicate four rows of detectors along the axial or z-direction, with each row including seven detector elements. In FIGURE 3, the array of detector elements of the flat detector **16$_f$** is represented by grid lines that indicate eight rows of detectors along the axial or z-direction, with each row including eleven detector elements. Those skilled in the art will recognize that existing flat and source-focused curved detector arrays typically have much larger numbers of detector elements, such as a dozen or more rows of detectors, with each row including dozens or more preferably hundreds of detector elements. In one preferred embodiment, the detector has more than one hundred rows of 0.75 mm$^2$ detector elements.

With returning reference to FIGURE 1, if an inexact image reconstruction that does not fulfill all the requirements of the three-dimensional Radon transform is acceptable, then a conventional parallel rebinned convolution backprojection can be employed. A parallel rebinning processor **34** rebins the filtered projection data into parallel views. A ramp convolution processor **36** performs one-dimensional convolution filtering to generate filtered projections. A three-dimensional parallel backprojector processor **42** receives the rebinned data and performs a three-dimensional parallel backprojection to obtain a reconstructed image representation that is stored in an image memory **44**.

Optionally, the filtered backprojector processor **42** includes weighting of the projection data with respect to detector position (aperture weighting) to reduce artifacts introduced by large projection components in the cone angle direction. The filtered backprojector processor **42** can also include weighting of the projection data with respect to the helix angle (angular weighting) to smooth the data at angular discontinuities, to combine angularly complementary projection data, or the like. Suitable three-dimensional filtered backprojector pipes are described, for example, in U.S. Patent No. 6,104,775 issued to Tuy, and in U.S. patent application serial no. 10/274,816 by Heuscher et al. filed on October 21, 2002.

The resultant image representation is suitably processed by a video processor **50** to generate a three-dimensional rendering, one or more image slices, or other visual representation of the reconstructed image that is displayed on a user interface **52**. Rather than a video display, the image representation can be formatted by a printer driver and printed out using a printer, transmitted over an electronic network, stored electronically, or otherwise utilized. Preferably, the user interface **52** communicates with a computed tomography controller **54** to enable a radiologist or other operator to initiate imaging or otherwise control operation of the computed tomography scanner **10**.

The inexact image reconstruction processing employing the parallel rebinning processor **34,** the ramp convolution processor **36,** and the parallel three-dimensional backprojector processor **42** may be adequate for certain applications. However, if an exact reconstruction which fulfills all the requirements of the three-dimensional Radon transform is desired, then an exact filtered backprojection conebeam reconstruction process with a one-dimensional shift-invariant filtering is employed. The disclosed exact reconstruction method is compatible with projection data that is axially or z-truncated. When performing exact conebeam reconstruction; a different processing path starting at a finite derivative processor **60** is engaged. That is, rather than inputting the projection data into the parallel rebinning processor **34,** the data is input into the finite derivative processor **60**.

With continuing reference to FIGURE 1, the one-dimensional shift-invariant filtering is performed by a hybrid convolution. A finite derivative processor **60** receives conebeam projection data from the projection data memory 30 and computes derivatives with respect to the helix angle $\lambda$ at fixed projection directions. A cone angle length correction processor **62** scales or normalizes lengths of the conebeam projections with respect to the source-to-detector distance D. The projection lengths vary across the curved detector **16$_c$** of FIGURE 2 and the flat detector **16$_f$** of FIGURE 3. Filtered projection data is computed from .the differentiated and length-normalized projection data using a one-dimensional convolution processor **64**. In a preferred embodiment, the convolution processor **64** includes a forward height rebinning processor **70,** an FFT convolution processor **72** that performs a one-dimensional Hilbert-based FFT convolution along the fan direction $\alpha$ (in the curved detector geometry) or u (in the flat detector geometry), and a reverse-height rebinning processor **74** that performs projection rebinning in the $\alpha$ or u direction.

A post-cosine weighting processor **80** performs a $\cos(\alpha)$ weighting in the case of the curved detector geometry of FIGURE 2. For the flat detector geometry of FIGURE 3, the $\cos(\alpha)$ weighting is preferably omitted. Omission of the $\cos(\alpha)$ weighting in the case of a flat detector geometry is optionally achieved by setting the weights applied by the weighting processor **80** to unity. The data output by the post-cosine weighting processor **80** in the case of a curved detector geometry, or output by the convolution processor **64** in the case of a flat detector geometry, is suitably input to a conebeam backprojection processor **82** which backprojects the filtered projection data to generate an image representation that is stored in the image memory **44**. The image representation is suitably processed by the video processor **50** and displayed on the user interface 52, or otherwise utilized.

Alternatively, the data output by the convolution processor **64** is input to an inverse cosine weighting processor **80',** parallel-rebinned by a parallel rebinning processor **34',** and backprojected by the 3D parallel backprojection processor **42** to generate an image representation that is stored in the image memory **44**. For the flat detector geometry of FIGURE 3, the inverse cosine weighting is suitably omitted. It will be appreciated that this alternative processing of the hybrid-convolved data advantageously can employ the same 3D parallel backprojection processor **42** used in the inexact reconstruction. Moreover, the parallel rebinning processor **34'** optionally employs the same physical hardware or software module as the parallel rebinning processor **34** of the inexact reconstruction.

Those skilled in the art will appreciate that the described hybrid convolution processing operates entirely within the native scan coordinates of the curved or flat detector **16$_c$, 16$_f$.** This increases reconstruction speed versus exact reconstruction performed in a detector independent coordinate system by eliminating computationally intensive rebinning operations associated with converting to the detector-independent voxel-based geometry. Moreover, reconstruction accuracy is promoted by avoiding interpolations involved in the rebinning to a voxel-based coordinate system.

The reconstruction processing components can be physically implemented in various ways. Some or all components can be software modules that are executed on one or more computers. Some or all components can be application-specific hardware pipeline components. Some or all of the reconstruction processing components can be integrated into the user interface **52,** and/or into the computed tomography scanner **10,** or can be stand-alone components. Hardware pipeline components are readily embodied as computer cards that insert into interface slots of a computer to enable integration of hardware- and software-based reconstruction processing components. Moreover, a given component, such as the backprojector **42, 82** can be implemented partially in software and partially as a hardware pipeline. Those skilled in the art can readily construct other physical implementations using other combinations of hardware and software.

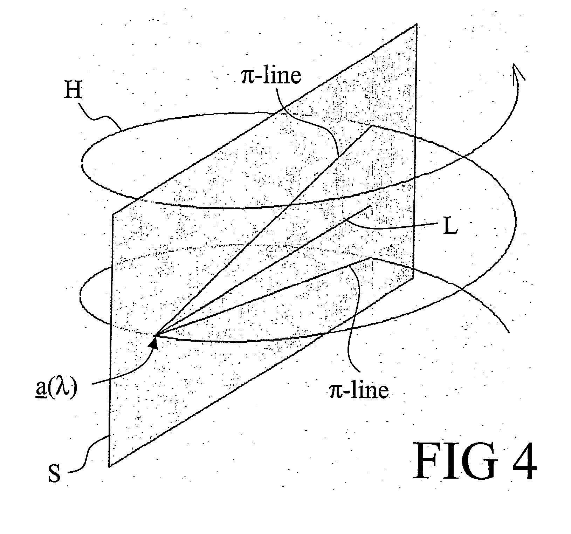

With reference to FIGURE 4, $\pi$-line and $\kappa$-plane geometrical constructs that are used in the exact conebeam reconstruction are described. A $\pi$-line is a line segment that connects two vertex points $\underline{a}(\lambda)$ separated by less than one pitch P in the axial or z-direction. (Equation (1) defines the pitch P). FIGURE 4 shows a helical trajectory H of the x-ray source **12** and a vertex point $\underline{a}(\lambda)$ of interest. All $\pi$-lines have the following property: for every point within the field of view, there is a unique $\pi$-line that passes through that point. Geometrically, a helix segment connecting the endpoints of a $\pi$-line define a 180° angular coverage for points along the $\pi$-line within the field of view. Axially truncated conebeam data acquired along a helix segment joining the endpoints of a $\pi$-line provide enough information for exact reconstruction of image points along the $\pi$-line.

A $\kappa$-plane is a plane that has three intersections with the helix such that the middle helix intersection point is equally angularly spaced in helix angle $\lambda$ from the two other helix intersection points. There exists a family of $\kappa$-planes corresponding to each vertex point $\underline{a}(\lambda)$. These corresponding $\kappa$-planes are indexed by an angle $\psi$ that lies in a range $(-\pi, \pi)$. The $\kappa$-plane of angle $\psi$ at the vertex point $\underline{a}(\lambda)$ is designated herein as $K(\lambda,\psi)$ and contains the vertex points $\underline{a}(\lambda)$, $\underline{a}(\lambda+\psi)$, and $\underline{a}(\lambda+2\psi)$. A unit vector normal to $K(\lambda,\psi)$ and having an acute angle with respect to the helix axis is designated herein as $\underline{n}(\lambda,\psi)$, and is given by:

$$\underline{n}(\lambda,\psi) = \frac{\underline{a}(\lambda+\psi) - \underline{a}(\lambda) \times \underline{a}(\lambda+2\psi) - \underline{a}(\lambda)}{\left\| \underline{a}(\lambda+\psi) - \underline{a}(\lambda) \times \underline{a}(\lambda+2\psi) - \underline{a}(\lambda) \right\|} sign(\psi) \qquad (2)$$

where the symbol "x" denotes a cross-product. As the angle $\psi$ tends toward zero, the $\kappa$-plane $K(\lambda,\psi)$ converges to a plane that is tangent to the helix H at $\underline{a}(\lambda)$ and is parallel to the detector v coordinate, that is:

$$\lim_{\psi \to 0} \underline{n}(\lambda,\psi) = \frac{1}{\sqrt{1 + 4\pi^2 R_o^2 / P^2}} \left[ \sin(\lambda + \lambda_0), -\cos(\lambda + \lambda_0), 2\pi R_o / P \right] \qquad (3).$$

All $\kappa$-planes have the following property, described with reference to FIGURE 4. A plane S is defined that intersects the field of view, is parallel to the helix axis, and contains $\underline{a}(\lambda)$. The plane S also contains two $\pi$-lines that connect the vertex point $\underline{a}(\lambda)$ with two other vertex points on the helix that also lie within the plane S. The $\kappa$-planes have the property that for any line L that contains $\underline{a}(\lambda)$ and that lies in the plane S between the two $\underline{a}(\lambda)$-terminating $\pi$-lines lying in the plane S, there exists a value of the angle $\psi$ in the range $(-\pi, \pi)$ at which the line L belongs to the $\kappa$-plane $K(\lambda,\psi)$. This property holds for any field of view less than the source distance $R_o$. There may be more than one $\kappa$-plane containing the line L.

With returning reference to FIGURE 1, the exact conebeam reconstruction method is described in the source-focused curved detector coordinates geometry of FIGURE 2. The finite derivative processor **60** computes derivative projection data $g_1(\lambda,\alpha,w)=g'(\lambda,\underline{\theta}_c)$ with respect to helix angle $\lambda$ at fixed projection direction vector $\underline{\theta}_c$. For the curved detector coordinate system, rotated coordinate axes $[\underline{e}_u(\lambda), \underline{e}_v(\lambda), \underline{e}_w]$ are defined with respect to the stationary coordinates $(x, y, z)$ in terms of the helix angle $\lambda$ according to:

$$\underline{e}_u(\lambda) = [-\sin(\lambda + \lambda_o), \cos(\lambda + \lambda_o), 0]$$

$$\underline{e}_v(\lambda) = [-\cos(\lambda + \lambda_o), -\sin(\lambda + \lambda_o), 0] \tag{4}$$

$$\underline{e}_w = [0, 0, 1]$$

where the directions u, v, w are set forth in Table I. In this rotated coordinate system and for the curved detector geometry of FIGURE 2, the projection direction vector $\underline{\theta}_c$ is given by:

$$\underline{\theta}_c = \frac{1}{\sqrt{D^2 + w^2}} \left( D \sin(\alpha)\, \underline{e}_u(\lambda) + D \cos(\alpha)\, \underline{e}_v(\lambda) + w\, \underline{e}_w \right) \tag{5}.$$

Conversely, given the projection direction vector $\underline{\theta}_c$ the curved detector coordinates $(\alpha, w)$ can be computed as:

$$\alpha = \tan^{-1}\left( \frac{\underline{\theta}_c \cdot \underline{e}_u(\lambda)}{\underline{\theta}_c \cdot \underline{e}_v(\lambda)} \right) \quad , \quad w = \frac{D\,(\underline{\theta}_c \cdot \underline{e}_w)}{\sqrt{1 - (\underline{\theta}_c \cdot \underline{e}_w)^2}} \tag{6},$$

where the dot operator denotes a dot product. Using these coordinates, the derivative of $g(\lambda, \alpha, w)$ with respect to helix angle $\lambda$ at fixed projection direction $\theta_c$ for the curved detector coordinate system is given by:

$$g_1(\lambda, \alpha, w) = g'(\lambda, \underline{\theta}_c) = \lim_{\varepsilon \to 0} \frac{g(\lambda + \varepsilon, \underline{\theta}_c) - g(\lambda, \underline{\theta}_c)}{\varepsilon} \tag{7}.$$

The derivative of Equation (7) is preferably implemented by the finite derivative processor **60** using a discrete finite difference derivative computation that arithmetically combines four neighboring projection samples. For an exemplary case where the sampling increment in coordinate $\alpha$ is one-fourth the sampling increment in the helix angle $\lambda$, that is, $\Delta\lambda = 4\Delta\alpha$, a suitable finite derivative is:

$$D'_{n,j} = a_0\, D_{n-1,j} + a_1\, D_{n,j} + a_2\, D_{n,j+1} + a_3\, D_{n+1,j+1} \tag{8}$$

where n is the discrete projection sample index along the helix angle direction $\lambda$, j is the discrete projection sample index along the $\alpha$ coordinate direction, and the constants $a_0$, $a_1$, $a_2$, $a_3$ are given by:

$$a_0 = 1/8$$

$$a_1 = 7/8 \tag{9},$$

$$a_2 = -7/8$$

$$a_3 = -1/8$$

A half-sample shift along the helix angle $\lambda$ is introduced by the finite derivative of Equations (8) and (9).

It will be recognized that the finite derivative of Equation (8) is advantageously performed on the projection data in the curved detector coordinate system without computationally intensive rebinning. Equations (8) and (9) are exemplary equations for the specific case of finite derivative computation using four neighboring projections with a spacing ratio of $\Delta\lambda = 4\Delta\alpha$. Those skilled in the art can readily construct other finite derivatives using another number of neighboring projections, for other spacing ratios, and so forth.

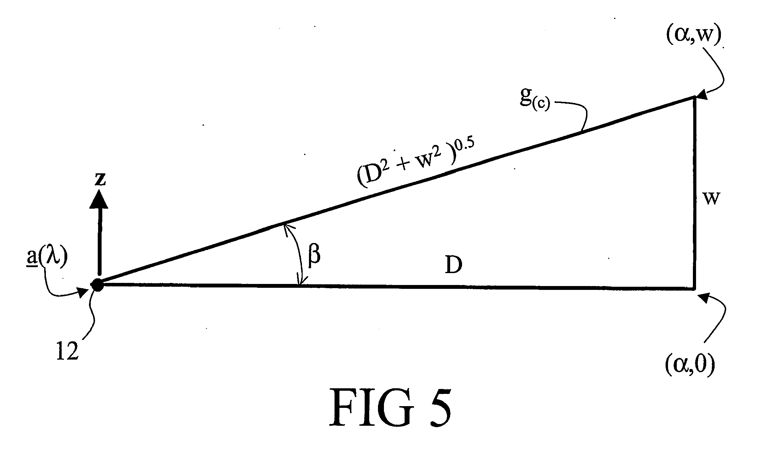

With reference to FIGURE 5, the differentiated projection data $g_1(\lambda,\alpha,w)=g'(\lambda,\underline{\theta}_c)$ output by the finite derivative processor **60** is processed by the cone angle length correction processor **62**. The length of a projection $g_{(c)}(\lambda,\alpha,w)$ for a given coordinate w in the cone angle direction is independent of the fan coordinate $\alpha$, due to the source-focused curvature of the detector **16c** along fan direction. However, as shown in FIGURE 5, a projection $g_{(c)}(\lambda,\alpha,w)$ has a projection length $(D^2+w^2)^{0.5}$. Accordingly, projections $g_{(c)}(\lambda,\alpha,0)$ have projection length D. Hence, the projection lengths for the source-focused curved detector **16c** are suitably normalized to the source-to-detector distance D by the operation:

$$g_2(\lambda,\alpha,w) = \cos(\beta)\, g_1(\lambda,\alpha,w) = \frac{D}{\sqrt{D^2 + w^2}}\, g_1(\lambda,\alpha,w) \tag{10}.$$

The differentiated and length-normalized projection data output by the cone angle length correction processor **62** is processed by the convolution processor **64.** Preferably, the convolution is implemented by the FFT convolution processor **72.** The convolution is a one-dimensional convolution with respect to coordinate $\alpha$ performed along intersections of $\kappa$-planes $K(\lambda,\psi)$ with the curved detector **16c.** In other words, the convolution is a one-dimensional convolution with respect to coordinate $\alpha$ performed at a fixed-angle $\psi$. Hence, prior to input to the FFT convolution processor **72,** the differentiated and length-normalized projection data is rebinned by the forward height rebinning processor **70** to get constant $\psi$ surfaces according to:

$$g_3(\lambda,\alpha,\psi) = g_2(\lambda,\alpha,w_\kappa(\alpha,\psi)) \tag{11},$$

over all $\psi$ in a range $[-\pi/2-\alpha_m, \pi/2+\alpha_m]$ where $\alpha_m$ is a fan angle defined by the size R of the field of view and the helix radius $R_o$, that is, $\alpha_m=\arcsin(R/R_o)$, with $w_\kappa(\alpha,\psi)$ given by:

$$w_\kappa(\alpha,\psi) = \frac{DP}{2\pi R_o}\left(\psi\cos(\alpha) + \frac{\psi}{\tan(\psi)}\sin(\alpha)\right) \tag{12}.$$

At a fixed angle $\psi$, it will be recognized that Equation (12) describes a curve in the detector area that is the intersection between the detector array of the curved detector **16c** and the $\kappa$-plane $K(\lambda,\psi)$. This curve is referred to herein as a $\kappa$-curve of angle $\psi$.

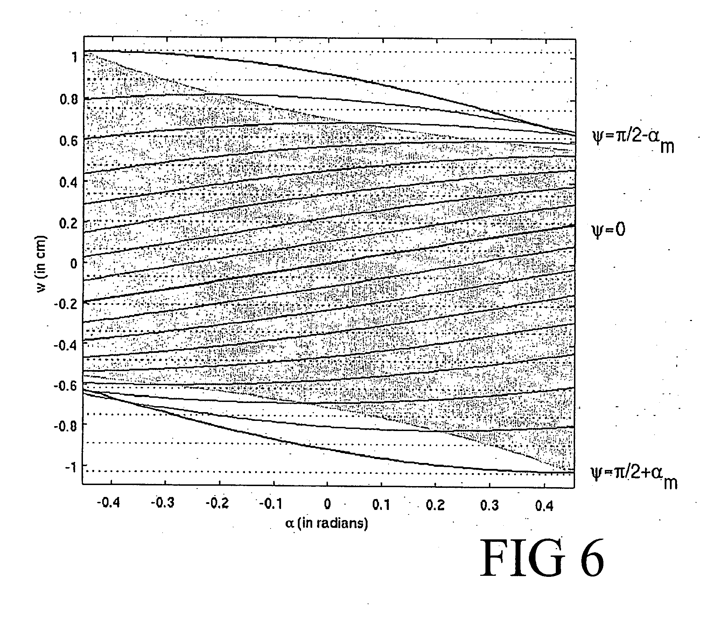

With reference to FIGURE 6, exemplary $\kappa$-curves for a source-focused curved detector panel with sixteen detector rows is shown. In FIGURE 6 the solid lines are the $\kappa$-curves, and a Tam-Danielsson window is shown as a shaded area. The Tam-Danielsson window will be referenced later.

The FFT convolution processor **72** is applied to the rebinned data $g_3(\lambda,\alpha,\psi)$ as a one-dimensional convolution in angle $\alpha$ for constant angle $\psi$ according to:

$$g_4(\lambda,\alpha,\psi) = \int_{-\pi/2}^{+\pi/2} d\alpha'\, h_H(\sin(\alpha-\alpha'))\, g_3(\lambda,\alpha',\psi) \tag{13}$$

where $h_H$ is a kernel based on a Hilbert transform. A suitable kernel $h_H$ is abs(sin(x))/sin(x) which in the s-domain is:

$$h_H(s) = -\int_{-\infty}^{+\infty} d\sigma \, i \, \text{sgn}(\sigma) \, e^{i2\pi\sigma s} = \frac{1}{\pi s} \qquad (14).$$

However, in view of the half-sample shift introduced by the selected finite derivative of Equations (8) and (9), a compensating half-sample shift is preferably incorporated into the kernel $h_H$.

After the one-dimensional convolution processor **72**, the projection data $g_4(\lambda,\alpha,\psi)$ is processed by the backward height rebinning processor **74** to obtain rebinned projection data $g_5(\lambda,\alpha,w)$ according to:

$$g_5(\lambda,\alpha,w) = g_4(\lambda,\alpha,\psi_{min}(\alpha,w)) \qquad (15),$$

where $\psi_{min}$ is the angle $\psi$ of smallest absolute value that satisfies the equation:

$$w = \frac{DP}{2\pi R}\left(\psi\cos(\alpha) + \frac{\psi}{\tan(\psi)}\sin(\alpha)\right) \qquad (16).$$

Reviewing the operation of the exemplary convolution processor **64**, it will be seen that the output $g_5(\lambda,\alpha,w)$ at a given detector location $(\alpha_o,w_o)$ is obtained by convolving projection data $g_2(\lambda,\alpha,w)$ on the $\kappa$-curve of smallest absolute $\psi$ value that passes through $(\alpha_o,w_o)$.

For processing data in the source-focused curved detector geometry, the output of the convolution processor **64** is preferably processed by the post-cosine weighting processor **80**. This weighting adjusts for the convolution in the fan direction according to:

$$g^F_{(c)}(\lambda,\alpha,w) = \cos(\alpha) \, g_5(\lambda,\alpha,w) \qquad (17)$$

to produce the final filtered data $g^F_{(c)}(\lambda,\alpha,w)$.

The filtered data $g^F_{(c)}$ is suitable for subsequent processing by a conebeam backprojector processor **82** to produce an exact reconstruction of conebeam projection data. In a suitable embodiment, the conebeam backprojector processor **82** computes the image $f(\underline{x})$ according to:

$$f(\underline{x}) = \frac{1}{2\pi}\int_{\lambda_i(\underline{x})}^{\lambda_o(\underline{x})} d\lambda \, \frac{1}{v^*(\lambda,\underline{x})} g^F_{(c)}\left(\lambda,\alpha^*(\lambda,\underline{x}),w^*(\lambda,\underline{x})\right) \qquad (18),$$

where $\lambda_i(\underline{x})$ and $\lambda_o(\underline{x})$ are the endpoints of the $\pi$-line through $\underline{x}$ with $\lambda_i(\underline{x})<\lambda_o(\underline{x})$, and:

$$v^*(\lambda,\underline{x}) = R - x\cos(\lambda+\lambda_o) - y\sin(\lambda+\lambda_o) \qquad (19),$$

$$\alpha * (\lambda, \underline{x}) = \arctan\left(\frac{1}{v*(\lambda, \underline{x})}\left(-x\sin(\lambda + \lambda_o) + y\cos(\lambda + \lambda_o)\right)\right) \qquad (20),$$

and

$$w*(\lambda, \underline{x}) = \frac{D\cos(\alpha*(\lambda, \underline{x}))}{v*(\lambda, \underline{x})}\left(z - z_o - \frac{P}{2\pi}(\lambda + \lambda_o)\right) \qquad (21).$$

The backprojection of Equations (18)-(21) implemented by the conebeam backprojector processor **82** is exemplary only. Those skilled in the art can employ other suitable backprojection processes. For instance, in an alternative backprojection path shown in FIGURE 1, the filtered projection data $g^F_{(c)}$ is input to the inverse cosine weighting processor **80'**, the parallel rebinning processor **34'**, and the parallel-rebinned filtered projection data is input to the 3D parallel backprojector **42** for backprojection. This latter pathway advantageously eliminates the computationally intensive $1/v$ weighting of the conebeam backprojector **82**. The inverse cosine weighting processor 80' applies a $l/\cos(\alpha)$ weighting. This different weighting as compared with the $\cos(\alpha)$ weighting applied by the cosine weighting processor **80** (see Equation 17) accounts for a difference in internal projection data weighting in the two backprojectors **42, 80**. A suitable 3D parallel backprojector **42** is described in U.S. patent application serial no. 10/274,816 by Heuscher et al., filed on October 21, 2002, which discloses a three-dimensional parallel backprojector pipeline that advantageously facilitates weighted combining of redundant projection data, that is, projection data for a given voxel that are separated in helix angle by integer multiples of 180°, to compensate for motion artifacts and other imaging imperfections.

In performing above-described exact reconstruction method, the number of detector rows should be large enough so that the filtered data can be computed for points of the region of interest. The number of detector rows, the helix pitch P, the helix radius $R_o$, and the size of the field of view can be interrelated. One example of a suitable detector region for conebeam reconstruction by the backprojection of Equation (18) is known in the art as the Tam-Danielsson window. An exemplary Tam-Danielsson window is shown as the shaded area on the detector in FIGURE 6. Referring again to the angle $\alpha_m$, where $\alpha_m$ is the fan angle defined by the size R of the field of view and the helix radius $R_o$, that is, $\alpha_m = \arcsin(R/R_o)$, the region of the Tam-Danielsson window is the set of $(\alpha, w)$ points such that $\alpha$ lies in a range $[-\alpha_m, \alpha_m]$, and w lies between a minimum value $w_{bottom}(\alpha)$ given by:

$$w_{bottom}(\alpha) = -\frac{DP}{2\pi R_o}\frac{\pi/2 + \alpha}{\cos(\alpha)} \qquad (22)$$

and a maximum value $w_{top}(\alpha)$ given by:

$$w_{top}(\alpha) = \frac{DP}{2\pi R_o}\frac{\pi/2 - \alpha}{\cos(\alpha)} \qquad (23).$$

The convolution processor **64** is a non-local process that uses projection data outside the Tam-Danielsson window. However, as shown in FIGURE 6 the number of detector rows needed to cover the Tam-Danielsson window generally also covers the range between the $\kappa$-curves $K(\lambda, \psi)$ in the range $-\pi/2-\alpha_m$, $\leq \psi \leq \pi/2+\alpha_m$. Hence, it is generally sufficient for the exact conebeam reconstruction to provide enough detector rows to span the range $[w_{bottom}, W_{top}]$. This number of detector rows is about:

$$N_{rows} = \frac{d_w + w_{top}(\alpha_m) - w_{bottom}(\alpha_m)}{d_w} = 1 + \frac{DP}{\pi R_o d_w}\frac{\pi/2 + \alpha_m}{\cos(\alpha_m)} \qquad (24),$$

where $d_w$ is a thickness of the detector rows.

The exact conebeam reconstruction method described with reference to Equation (4) through Equation (24) is suitable for reconstructing projection data formatted in the source-centered curved detector geometry of FIGURE 2. Reconstruction of projection data formatted in the flat detector geometry of FIGURE 3 is described next.

With reference to FIGURES 1 and 3, the flat detector array **16$_f$** is parallel to the helix axis, that is, parallel to the z-direction. The orientation of the detector array **16$_f$** changes with helical movement of the vertex point $\underline{a}(\lambda)$, that is, with the helix angle $\lambda$, so as to remain orthogonal to a plane containing both the vertex point $\underline{a}(\lambda)$ and the helix axis. As shown in FIGURE 1, this is suitably accomplished by mounting the detector **16$_f$** opposite the x-ray source **12** on the rotating gantry **22.** Alternatively, the flat detector can be mounted as a detector ring on the stationary gantry **24.** Similarly to the case of the curved detector, the source-to-detector distance D corresponds to a distance from the vertex point $\underline{a}(\lambda)$ to the plane of the flat detector **16$_f$**, as indicated in FIGURE 3. The rotated coordinate axes [$\underline{e}_u$, $\underline{e}_v$, $\underline{e}_w$] of Equation (4) apply to the flat detector geometry as well.

The projection direction vector $\underline{\theta}_f$ for the flat detector geometry is given by:

$$\underline{\theta}_f = \frac{1}{\sqrt{u^2 + D^2 + w^2}}\left(u\,\underline{e}_u(\lambda) + D\,\underline{e}_v(\lambda) + w\,\underline{e}_w\right) \qquad (25).$$

Conversely, given the projection direction vector $\underline{\theta}_f$ the flat detector coordinates (u,w) can be computed as:

$$u = D\frac{\underline{\theta}_f \cdot \underline{e}_u(\lambda)}{\underline{\theta}_f \cdot \underline{e}_v(\lambda)} \qquad , \qquad w = D\frac{\underline{\theta}_f \cdot \underline{e}_w}{\underline{\theta}_f \cdot \underline{e}_v(\lambda)} \qquad (26),$$

where the dot operator denotes a dot product. Using these coordinates, the derivative of projection data g($\lambda$,u,w) for the flat detector coordinate system with respect to helix angle $\lambda$ at fixed projection direction $\underline{\theta}_f$ is computed by the finite derivative processor **60** in accordance with Equations (7)-(9), making the substitutions of $\underline{\theta}_f$ for $\underline{\theta}_c$ and u for $\alpha$ in Equation (7), to obtain differentiated projection data g$_1$($\lambda$,u,w)=g'($\theta$,$\underline{\theta}_f$).

The length correction in the case of the flat detector geometry is computed by the cone angle length correction processor 62 as:

$$g_2(\lambda,u,w) = \frac{D}{\sqrt{u^2 + D^2 + w^2}}\,g_1(\lambda,u,w) \qquad (27),$$

which is analogous to the normalization of Equation (10) for the curved detector geometry.

The forward height rebinning processor **70** performs rebinning of the flat detector geometry projection data according to:

$$g_3(\lambda,u,\psi) = g_2(\lambda,u,w_\kappa(u,\psi)) \qquad (28),$$

where $w_\kappa(u,\psi)$ is:

$$w_\kappa(u,\psi) = \frac{DP}{2\pi R_o}\left(\psi + \frac{\psi}{\tan(\psi)}\frac{u}{D}\right) \qquad (29).$$

Equations (28) and (29) are analogous to Equations (11) and (12) of the curved detector geometry processing. Analogously to the case of the curved detector geometry, Equation (29) defines $\kappa$-curves $K(\lambda,\psi)$ in the flat detector geometry that correspond to intersections of $\kappa$-planes with the flat detector $16_f$.

The one-dimensional convolution processor **72** performs the following one-dimensional convolution with respect to coordinate u at constant angle $\psi$:

$$g_4(\lambda,u,\psi) = \int_{-\infty}^{+\infty} du'\, h_H(u-u')\, g_3(\lambda,u',\psi) \qquad (30)$$

where a suitable kernel $h_H$ is given in Equation (14). The reverse height rebinning processor **74** rebins the convolved projection data $g_4(\lambda,u,\psi)$ to produce filtered projection data $g^F{}_{(f)}(\lambda,u,w)$ according to:

$$g^F(\lambda,u,w) = g_4(\lambda,u,\psi_{min}(u,w)) \qquad (31),$$

where $\psi_{min}$ is the angle of smallest absolute value that satisfies:

$$w = \frac{DP}{2\pi R}\left(\psi + \frac{\psi}{\tan(\psi)}\frac{u}{D}\right) \qquad (32).$$

For the flat detector geometry, the post cosine weighting processor **80** or the inverse cosine weighting processor **80'** is bypassed, and so the output of the reverse height rebinning processor **74** is the filtered projection data $g^F{}_{(f)}(\lambda,u,w)$. It will be appreciated that the processes set forth in Equations (28)-(32) obtain $g^F{}_{(f)}(\lambda,u_o,w_o)$ from the projection data $g_2(\lambda,u,w)$ by convolving along the $\kappa$-curve of smallest absolute angle $\psi$ that passes through $(u_o,w_o)$.

The filtered data $g^F{}_{(f)}$ is suitable for subsequent processing by the conebeam backprojector processor **82** to produce an exact reconstruction of conebeam projection data. In a suitable embodiment, the conebeam backprojector processor **82** computes the image $f(\underline{x})$ according to:

$$f(\underline{x}) = \frac{1}{2\pi}\int_{\lambda_i(\underline{x})}^{\lambda_o(\underline{x})} d\lambda\, \frac{1}{v^*(\lambda,\underline{x})}\, g^F_{(f)}\big(\lambda, u^*(\lambda,\underline{x}), w^*(\lambda,\underline{x})\big) \qquad (33),$$

analogous to the backprojecting of the filtered projection data $g^F{}_{(c)}$ of the curved coordinate geometry given in Equation (18). The parameter $v^*(\lambda,\underline{x})$ is the same as that given in Equation (19) for the curved detector geometry, while the parameters $u^*(\lambda,\underline{x})$ and $w^*(\lambda,\underline{x})$ are given in the flat detector geometry by:

$$u^*(\lambda,\underline{x}) = \frac{D}{v^*(\lambda,\underline{x})}\left(-x\sin(\lambda+\lambda_o)+y\cos(\lambda+\lambda_o)\right) \tag{34},$$

and

$$w^*(\lambda,\underline{x}) = \frac{D}{v^*(\lambda,\underline{x})}\left(z-z_o-\frac{P}{2\pi}(\lambda+\lambda_o)\right) \tag{35}.$$

As in the case of backprojecting the filtered projection data $g^F_{(c)}$ of the curved coordinate geometry, the backprojection process of Equations (19) and (33)-(35) is exemplary only. In one alternative backprojection approach, the filtered backprojection data $g^F_{(f)}$ is input into the parallel rebinning processor **34'**, and the parallel-rebinned filtered backprojection data is backprojected using the 3D parallel backprojector **42**.

In the case of the flat detector geometry, a suitable number of detector rows $N_{rows}$ is estimated as follows. The Tam-Danielsson window is the set of (u,w) points with u in a range $[-u_m,u_m]$ where $u_m=D\tan(\alpha_m)=D\tan(\arcsin(R/R_o))$, and w in a range $[W_{bottom}(u),w_{top}(u)]$, where:

$$w_{bottom}(u) = -\frac{P}{2\pi R_o D}(u^2+D^2)\left(\pi/2+\arctan\left(\frac{u}{D}\right)\right) \tag{36},$$

and

$$w_{top}(u) = -\frac{P}{2\pi R_o D}(u^2+D^2)\left(\pi/2-\arctan\left(\frac{u}{D}\right)\right) \tag{37}.$$

Computation of the filtered data at any point in the Tam-Danielsson window employs data on $\kappa$-curves that intersect the Tam-Danielsson window. This data generally corresponds to data acquired using detector rows between $u_{bottom}$ and $u_{top}$ inclusive, and so a suitable number of detector rows is about:

$$N_{rows} = \frac{d_w+w_{top}(-u_m)-w_{bottom}(-u_m)}{d_w} \tag{38},$$

which can be rewritten as:

$$N_{rows} = 1+\frac{P}{\pi R_o D d_w}(u_m^2+D^2)\left(\frac{\pi}{2}+\arctan\left(\frac{u_m}{D}\right)\right) \tag{39},$$

where $d_w$ is the thickness of the detector rows.

The exact nature of the exact reconstructions described herein has been demonstrated by showing equivalency of image representations reconstructed using the techniques disclosed herein and image representations reconstructed using the exact method of Katsevich. The exact reconstruction methods described herein advantageously are performed entirely within the flat or curved detector coordinate system. Computationally intensive transformations of projection data into a detector-independent coordinate system is thus obviated, providing a more rapid reconstruction. The exact reconstruction methods described herein optionally incorporate the three-dimensional backprojector **42.** This enables the exact reconstruction to take advantage of three-dimensional backprojector capabilities such as complementary combination of redundant projection data..

The invention has been described with reference to the preferred embodiments. Obviously, modifications and alterations will occur to others upon reading and understanding the preceding detailed description. It is intended that the invention be construed as including all such modifications and alterations insofar as they come within the scope of the appended claims or the equivalents thereof.

**Claims**

1. A conebeam computed tomography imaging system including:

   an x-ray source (12) that produces an x-ray conebeam directed into an examination region (14), the x-ray source (12) being arranged to traverse a generally helical trajectory around the examination region (14) ;
   an x-ray detector array (16) arranged to detect the x-ray conebeam after passing through the examination region (14), the x-ray detector array (16) generating projection data in native scan coordinates defined with reference to the detector array (16); and
   an exact reconstruction processor (34', 42, 60, 62, 64, 80, 80', 82) that performs an exact reconstructing of conebeam projection data produced by the detector array (16) into an image representation;

   **characterized in that** the reconstructing is performed in the native scan coordinates;
   wherein the reconstruction processor (34', 42, 60, 62, 64, 80, 80', 82) includes:

   a derivative processor (60) that computes a derivative of the projection data with respect to a helix angle of the helical trajectory at fixed projection direction to generate differentiated projection data;
   a convolution processor (64) that convolves the differentiated projection data with a kernel function to produce filtered projection data, the convolving being performed in the native scan coordinates; and
   a backprojector (42, 82) that backprojects the filtered projection data to obtain an image representation.

2. The conebeam computed tomography imaging system as set forth in claim 1, wherein the convolving performed by the convolution processor (64) is a one-dimensional convolving employing a kernel function based on a Hilbert transform.

3. The conebeam computed tomography imaging system as set forth in claim 1, wherein the derivative processor 60 computes the derivative using a discrete finite difference derivative computation that arithmetically combines selected neighboring projection data.

4. The conebeam computed tomography imaging system as set forth in claim 1, wherein the reconstruction processor (34', 42, 60, 62, 64, 80, 80', 82) further includes:

   a cone angle length correction processor (62) that normalizes a length of each projection to a source-to-detector distance (D).

5. The conebeam computed tomography imaging system as set forth in claim 1, wherein the reconstruction processor (34', 42, 60, 62, 64, 80, 80', 82) further includes:

   a parallel rebinning processor (34') that parallel-rebins the filtered projection data, the backprojector (42) being a parallel backprojector that backprojects the parallel rebinned filtered backprojection data.

6. The conebeam computed tomography imaging system as set forth in claim 1, wherein the detector array ($16_c$) has a curved detector geometry generally focused on the x-ray source, and the detector coordinate system is a curved detector coordinate system having native scan coordinates including a helix angle coordinate ($\lambda$), a projection fan coordinate ($\alpha$), and a projection cone coordinate ($\beta$,w).

7. The conebeam computed tomography imaging system as set forth in claim 6, wherein the convolution processor (64) includes:

a first rebinning processor (70) that rebins the differentiated projection data along κ-curves to produce ψ-rebinned projection data where ψ indicates the κ-curve; and

a one-dimensional convolution transform processor (72) that one-dimensionally convolves the ψ-rebinned projection data with the kernel function to produce convolved projection data, the one dimensional convolving being in the projection fan coordinate (α) at constant ψ.

8. The conebeam computed tomography imaging system as set forth in claim 7, wherein the one dimensional convolution transform processor (72) performs the one-dimensional convolution according to:

$$g_{conv}(\lambda,\alpha,\psi) = \int_{-\pi/2}^{+\pi/2} d\alpha' \, h_H(\sin(\alpha - \alpha')) \, g_{rebin1}(\lambda,\alpha',\psi)$$

where $g_{rebin1}(\lambda,\alpha,\psi)$ indicates the first rebinned data, $h_H()$ indicates the kernel function, and $g_{conv}(\lambda,\alpha,\psi)$ indicates the convolved projection data.

9. The conebeam computed tomography imaging system as set forth in claim 7, wherein the convolution processor (64) further includes:

a weighting processor (80, 80') that weights the convolved projection data by one of a cosine of the projection fan coordinate (α) and an inverse cosine of the projection fan coordinate (α) to produce the filtered projection data.

10. The conebeam computed tomography imaging system as set forth in claim 9, wherein the backprojector (42, 82) includes:

a conebeam backprojector (82) that applies a 1/v weighting to the filtered projection data during the backprojecting.

11. The conebeam computed tomography imaging system as set forth in claim 6, wherein the reconstruction processor (34', 42, 60, 62, 64, 80, 80', 82) further includes:

a cone angle length correction processor (62) that scales projections by a cosine of the projection cone coordinate (β).

12. The conebeam computed tomography imaging system as set forth in claim 1, wherein the detector array ($16_f$) has a substantially flat detector geometry, and the detector coordinate system is a flat detector coordinate system having native scan coordinates including a helix angle coordinate (λ), a projection fan detector coordinate (u), and a projection cone coordinate (w).

13. The conebeam computed tomography imaging system as set forth in claim 12, wherein the convolution processor (64) includes:

a first rebinning processor (70) that rebins the differentiated projection data along κ-curves to produce ψ-rebinned projection data where ψ indicates the κ-curve; and

a one-dimensional convolution transform processor (72) that one-dimensionally convolves the ψ-rebinned projection data with the kernel function to produce convolved projection data, the one dimensional convolving being in the projection fan coordinate (u) at constant ψ.

14. The conebeam computed tomography imaging system as set forth in claim 13, wherein the one dimensional convolution transform processor (72) performs the one-dimensional convolution according to:

$$g_{conv}(\lambda,u,\psi) = \int_{-\infty}^{+\infty} du' \, h_H(\sin(u - u')) \, g_{rebin1}(\lambda,u',\psi)$$

where $g_{rebin1}(\lambda,u,\psi)$ indicates the first rebinned data, $h_H()$ indicates the kernel function, and $g_{conv}(\lambda,u,\psi)$ indicates

EP 1 599 836 B1

the convolved projection data.

15. The conebeam computed tomography imaging system as set forth in claim 13, wherein the convolution processor (64) further includes:

a reverse height rebinning processor (74) that rebins the convolved projection data.

16. The conebeam computed tomography imaging system as set forth in claim 1, wherein the detector array ($16_c$) has a curved detector geometry generally focused on the x-ray source, and the detector coordinate system is a curved detector coordinate system having native scan coordinates including a helix angle coordinate ($\lambda$), a projection fan coordinate ($\alpha$), and a projection cone coordinate ($\beta$,w).

17. The conebeam computed tomography imaging system as set forth in claim 1, wherein the detector array ($16_f$) has a substantially flat detector geometry, and the detector coordinate system is a flat detector coordinate system having native scan coordinates including a helix angle coordinate ($\lambda$), a projection fan detector coordinate (u), and a projection cone coordinate (w).

18. The conebeam computed tomography imaging system as set forth in claim 1, wherein the reconstruction processor (34', 42, 60, 62, 64, 80, 80', 82) includes:

a finite derivative processor (60) that computes a finite derivative of the projection data in the native scan coordinates along a first direction to generate differentiated projection data;
a convolution processor (64) that performs a one-dimensional convolution of the differentiated projection data in the native scan coordinates along a second direction that is different from the first direction to produce filtered projection data; and
a backprojector (42, 82) that backprojects the filtered projection data to obtain an image representation.

19. The conebeam computed tomography imaging system as set forth in claim 1, wherein the native scan coordinates include at least a helix angle ($\lambda$), a projection fan coordinate ($\alpha$,u), and a projection cone coordinate ($\beta$,w).

20. An exact reconstruction method for reconstructing conebeam computed tomography projection data having native scan coordinates that include at least a helix angle ($\lambda$), a projection fan coordinate ($\alpha$,u), and a projection cone coordinate ($\beta$,w);
**characterized in that** the method is performed in the native scan coordinates; wherein the method includes:

computing filtered projection data by a combination of:

computing a derivative of projection data with respect to the helix angle ($\lambda$) at fixed projection direction, and convolving projection data with a kernel function, the convolving being performed in the native scan coordinates; and
backprojecting the filtered projection data to obtain an image representation.

21. The method as set forth in claim 20, wherein the computing of a derivative includes:

computing each differentiated projection datum by a finite difference derivative based on a plurality of projection data that neighbor said differentiated projection datum.

22. The method as set forth in claim 20, wherein the convolving includes:

rebinning projection data with respect to $\kappa$-planes $K(\lambda,\psi)$ to group projections of the projection data by $\kappa$-plane coordinate $\psi$; and
one-dimensionally convolving the rebinned projection data with the kernel function, the one-dimensional convolving being with respect to the projection fan coordinate ($\alpha$,u), the one-dimensional convolving being at fixed $\kappa$-plane coordinate $\psi$.

23. The method as set forth in claim 20, wherein the backprojecting includes:

parallel rebinning the filtered projection data to obtain parallel projection views; and

16

backprojecting the parallel projection views.

24. The method as set forth in claim 23, wherein the native scan coordinates have a curved geometry, and the method further includes:

prior to the backprojecting, weighting the filtered projection data by an inverse cosine of the projection fan coordinate ($\alpha$).

25. The method as set forth in claim 20, wherein the backprojecting includes:

weighting the filtered projection data by a 1/v factor; and
backprojecting the filtered data with the 1/v weighting.

26. The method as set forth in claim 25, wherein the native scan coordinates have a curved geometry, and the method further includes:

prior to the backprojecting, weighting the filtered projection data by a cosine of the projection fan coordinate ($\alpha$).

27. The method as set forth in claim 20, wherein the kernel function is based on a Hilbert transform.

28. The method as set forth in claim 20, wherein the computing of filtered projection data further includes:

normalizing a projection length of projection data.

**Patentansprüche**

1. Kegelstrahl-Computertomographie-Bildgebungssystem, das Folgendes umfasst:

eine Röntgenquelle (12), die ein auf eine Untersuchungsregion (14) gerichtetes Röntgenkegelstrahlenbündel erzeugt, wobei die Röntgenquelle (12) vorgesehen ist, um eine im Allgemeinen spiralförmige Trajektorie um die Untersuchungsregion (14) zu durchlaufen;
ein Röntgendetektorarray (16), das vorgesehen ist, um das Röntgenkegelstrahlenbündel nach dem Durchqueren der Untersuchungsregion (14) zu detektieren, wobei das Röntgendetektorarray (16) Projektionsdaten in nativen Scan-Koordinaten erzeugt, die in Bezug auf das Detektorarray (16) definiert sind; und
einen exakten Rekonstruktionsprozessor (34', 42, 60, 62, 64, 80, 80', 82), der eine exakte Rekonstruktion der durch das Detektorarray (16) erzeugten Kegelstrahl-Projektionsdaten zu einer Bilddarstellung durchführt;

**dadurch gekennzeichnet, dass** die Rekonstruktion in den nativen Scan-Koordinaten durchgeführt wird;
wobei der Rekonstruktionsprozessor (34', 42, 60, 62, 64, 80, 80', 82) Folgendes umfasst:

einen Ableitungsprozessor (60), der eine Ableitung der Projektionsdaten in Bezug auf einen Helixwinkel der spiralförmigen Trajektorie bei fester Projektionsrichtung berechnet, um differenzierte Projektionsdaten zu erzeugen;
einen Faltungsprozessor (64), der die differenzierten Projektionsdaten mit einer Kernel-Funktion faltet, um gefilterte Projektionsdaten zu erzeugen, wobei das Falten in den nativen Scan-Koordinaten durchgeführt wird; und
einen Rückprojektor (42, 82), der die gefilterten Projektionsdaten rückprojiziert, um eine Bilddarstellung zu erhalten.

2. Kegelstrahl-Computertomographie-Bildgebungssystem nach Anspruch 1, wobei das durch den Faltungsprozessor (64) durchgeführte Falten ein eindimensionales Falten unter Verwendung einer Kernel-Funktion basierend auf einer Hilbert-Transformation ist.

3. Kegelstrahl-Computertomographie-Bildgebungssystem nach Anspruch 1, wobei der Ableitungsprozessor 60 die Ableitung unter Verwendung einer diskreten finiten Differenzableitungberechnung berechnet, die ausgewählte benachbarte Projektionsdaten arithmetisch kombiniert.

4. Kegelstrahl-Computertomographie-Bildgebungssystem nach Anspruch 1, wobei der Rekonstruktionsprozessor (34', 42, 60, 62, 64, 80, 80', 82) weiterhin Folgendes umfasst:

einen Kegelwinkel-Längenkorrekturprozessor (62), der eine Länge jeder Projektion auf einen Quellen-Detektor-Abstand (D) normalisiert.

5. Kegelstrahl-Computertomographie-Bildgebungssystem nach Anspruch 1, wobei der Rekonstruktionsprozessor (34', 42, 60, 62, 64, 80, 80', 82) weiterhin Folgendes umfasst:

einen Parallel-Rebinning-Prozessor (34'), der die gefilterten Projektionsdaten dem Parallel-Rebinning unterzieht, wobei der Rückprojektor (42) ein paralleler Rückprojektor ist, der die dem Parallel-Rebinning unterzogenen gefilterten Rückprojektionsdaten rückprojiziert.

6. Kegelstrahl-Computertomographie-Bildgebungssystem nach Anspruch 1, wobei das Detektorarray ($16_c$) eine gekrümmte Detektorgeometrie hat, die im Allgemeinen auf die Röntgenquelle fokussiert ist, und das Detektorkoordinatensystem ein gekrümmtes Detektorkoordinatensystem mit nativen Scan-Koordinaten einschließlich einer Helixwinkelkoordinate ($\lambda$), einer Projektionsfächerkoordinate ($\alpha$) und einer Projektionskegelkoordinate ($\beta$, w) ist.

7. Kegelstrahl-Computertomographie-Bildgebungssystem nach Anspruch 6, wobei der Faltungsprozessor (64) Folgendes umfasst:

einen ersten Rebinning-Prozessor (70), der die differenzierten Projektionsdaten entlang $\kappa$-Kurven einem Rebinning unterzieht, um dem $\psi$ -Rebinning unterzogene Projektionsdaten zu erzeugen, wobei $\psi$ die $\kappa$-Kurve angibt; und
einen eindimensionalen Faltungstransformationsprozessor (72), der die dem $\psi$-Rebinning unterzogenen Projektionsdaten mit der Kernel-Funktion eindimensional faltet, um gefaltete Projektionsdaten zu erzeugen, wobei das eindimensionale Falten in der Projektionsfächerkoordinate ($\alpha$) bei konstantem $\psi$ erfolgt.

8. Kegelstrahl-Computertomographie-Bildgebungssystem nach Anspruch 7, wobei der eindimensionale Faltungstransformationsprozessor (72) die eindimensionale Faltung gemäß folgender Gleichung durchführt:

$$g_{falt}(\lambda,\alpha,\psi) = \int\limits_{-\pi/2}^{+\pi/2} d\alpha' \, h_H\left(\sin\left(\alpha-\alpha'\right)\right) g_{rebin1}(\lambda,\alpha',\psi)$$

wobei $g_{rebin1}(\lambda, \alpha, \psi)$ die ersten dem Rebinning unterzogenen Daten bezeichnet, $h_H()$ die Kernel-Funktion bezeichnet und $g_{falt}(\lambda, \alpha, \psi)$ die gefalteten Projektionsdaten bezeichnet.

9. Kegelstrahl-Computertomographie-Bildgebungssystem nach Anspruch 7, wobei der Faltungsprozessor (64) weiterhin Folgendes umfasst:

einen Gewichtungsprozessor (80, 80'), der die gefalteten Projektionsdaten durch entweder einen Kosinus der Projektionsfächerkoordinate ($\alpha$) oder einen inversen Kosinus der Projektionsfächerkoordinate ($\alpha$) gewichtet, um die gefilterten Projektionsdaten zu erzeugen.

10. Kegelstrahl-Computertomographie-Bildgebungssystem nach Anspruch 9, wobei der Rückprojektor (42, 82) Folgendes umfasst:

einen Kegelstrahl-Rückprojektor (82), der während der Rückprojektion eine 1/v-Gewichtung auf die gefilterten Projektionsdaten anwendet.

11. Kegelstrahl-Computertomographie-Bildgebungssystem nach Anspruch 6, wobei der Rekonstruktionsprozessor (34', 42, 60, 62, 64, 80, 80', 82) weiterhin Folgendes umfasst:

einen Kegelwinkel-Längenkorrekturprozessor (62), der Projektionen durch einen Kosinus der Projektionskegelkoordinate ($\beta$) skaliert.

**12.** Kegelstrahl-Computertomographie-Bildgebungssystem nach Anspruch 1, wobei das Detektorarray ($16_f$) eine im Wesentlichen flache Detektorgeometrie hat, und das Detektorkoordinatensystem ein Flachdetektorkoordinatensystem mit nativen Scan-Koordinaten einschließlich einer Helixwinkelkoordinate ($\lambda$), einer Projektionsfächerdetektorkoordinate (u) und einer Projektionskegelkoordinate (w) ist.

**13.** Kegelstrahl-Computertomographie-Bildgebungssystem nach Anspruch 12, wobei der Faltungsprozessor (64) Folgendes umfasst:

einen ersten Rebinning-Prozessor (70), der die differenzierten Projektionsdaten entlang $\kappa$-Kurven einem Rebinning unterzieht, um dem $\psi$-Rebinning unterzogene Projektionsdaten zu erzeugen, wobei $\psi$ die $\kappa$-Kurve angibt; und
einen eindimensionalen Faltungstransformationsprozessor (72), der die dem $\psi$-Rebinning unterzogenen Projektionsdaten mit der Kernel-Funktion eindimensional faltet, um gefaltete Projektionsdaten zu erzeugen, wobei das eindimensionale Falten in der Projektionsfächerkoordinate (u) bei konstantem $\psi$ erfolgt.

**14.** Kegelstrahl-Computertomographie-Bildgebungssystem nach Anspruch 13, wobei der eindimensionale Faltungstransformationsprozessor (72) die eindimensionale Faltung gemäß folgender Gleichung durchführt:

$$g_{falt}(\lambda, u, \psi) = \int\limits_{-\infty}^{+\infty} du'\, h_H\,(\sin\,(u-u'))\, g_{rebin1}(\lambda, u', \psi)$$

wobei $g_{rebin\,1}(\lambda, u, \psi)$ die ersten dem Rebinning unterzogenen Daten bezeichnet, $h_H()$ die Kernel-Funktion bezeichnet und $g_{falt}(\lambda, u, \psi)$ die gefalteten Projektionsdaten bezeichnet.

**15.** Kegelstrahl-Computertomographie-Bildgebungssystem nach Anspruch 13, wobei der Faltungsprozessor (64) weiterhin Folgendes umfasst:

einen Umkehrhöhen-Rebinning-Prozessor (74), der die gefalteten Projektionsdaten dem Rebinning unterzieht.

**16.** Kegelstrahl-Computertomographie-Bildgebungssystem nach Anspruch 1, wobei das Detektorarray ($16_c$) eine gekrümmte Detektorgeometrie hat, die im Allgemeinen auf die Röntgenquelle fokussiert ist, und das Detektorkoordinatensystem ein gekrümmtes Detektorkoordinatensystem mit nativen Scan-Koordinaten einschließlich einer Helixwinkelkoordinate ($\lambda$), einer Projektionsfächerkoordinate ($\alpha$) und einer Projektionskegelkoordinate ($\beta$, w) ist.

**17.** Kegelstrahl-Computertomographie-Bildgebungssystem nach Anspruch 1, wobei das Detektorarray ($16_f$) eine im Wesentlichen flache Detektorgeometrie hat, und das Detektorkoordinatensystem ein Flachdetektorkoordinatensystem mit nativen Scan-Koordinaten einschließlich einer Helixwinkelkoordinate ($\lambda$), einer Projektionsfächerdetektorkoordinate (u) und einer Projektionskegelkoordinate (w) ist.

**18.** Kegelstrahl-Computertomographie-Bildgebungssystem nach Anspruch 1, wobei der Rekonstruktionsprozessor (34', 42, 60, 62, 64, 80, 80', 82) Folgendes umfasst:

einen finiten Ableitungsprozessor (60), der eine finite Ableitung der Projektionsdaten in den nativen Scan-Koordinaten entlang einer ersten Richtung berechnet, um differenzierte Projektionsdaten zu erzeugen;
einen Faltungsprozessor (64), der eine eindimensionale Faltung der differenzierten Projektionsdaten in den nativen Scan-Koordinaten entlang einer zweiten Richtung durchführt, die sich von der ersten Richtung unterscheidet, um gefilterte Projektionsdaten zu erzeugen; und
einen Rückprojektor (42, 82), der die gefilterten Projektionsdaten rückprojiziert, um eine Bilddarstellung zu erhalten.

**19.** Kegelstrahl-Computertomographie-Bildgebungssystem nach Anspruch 1, wobei die nativen Scan-Koordinaten mindestens einen Helixwinkel eine Projektionsfächerkoordinate ($\alpha$, u) und eine Projektionskegelkoordinate ($\beta$, w) umfassen.

**20.** Exaktes Rekonstruktionsverfahren zur Rekonstruktion von Kegelstrahl-Computertomographie-Projektionsdaten mit nativen Scan-Koordinaten, die mindestens einen Helixwinkel (λ), eine Projektionsfächerkoordinate (α, u) und eine Projektionskegelkoordinate (β, w) umfassen;
**dadurch gekennzeichnet, dass** das Verfahren in den nativen Scan-Koordinaten durchgeführt wird; wobei das Verfahren Folgendes umfasst:

Berechnen von gefilterten Projektionsdaten durch eine Kombination der folgenden Schritte:

Berechnen einer Ableitung von Projektionsdaten in Bezug auf den Helixwinkel (λ) bei fester Projektionsrichtung, und
Falten der Projektionsdaten mit einer Kernel-Funktion, wobei das Falten in den nativen Scan-Koordinaten durchgeführt wird; und
Rückprojizieren der gefilterten Projektionsdaten, um eine Bilddarstellung zu erhalten.

21. Verfahren nach Anspruch 20, wobei das Berechnen der Ableitung Folgendes umfasst:

Berechnen jedes differenzierten Projektionsdatenwerts durch eine finite Differenzableitung basierend auf einer Vielzahl von Projektionsdaten, die dem genannten differenzierten Projektionsdatenwert benachbart sind.

22. Verfahren nach Anspruch 20, wobei das Falten Folgendes umfasst:

Rebinning der Projektionsdaten in Bezug auf K-Ebenen K (λ,ψ), um Projektionen von Projektionsdaten nach κ-Ebenen-Koordinate ψ zu gruppieren; und
eindimensionales Falten der dem Rebinning unterzogenen Projektionsdaten mit der Kernel-Funktion, wobei das eindimensionale Falten in Bezug auf die Projektionsfächerkoordinate (α, u) erfolgt, wobei das eindimensionale Falten bei fester κ-Ebenen-Koordinate ψ erfolgt.

23. Verfahren nach Anspruch 20, wobei das Rückprojizieren Folgendes umfasst:

paralleles Rebinning der gefilterten Projektionsdaten, um parallele Projektionsansichten zu erhalten; und
Rückprojizieren der parallelen Projektionsansichten.

24. Verfahren nach Anspruch 23, wobei die nativen Scan-Koordinaten eine gekrümmte Geometrie haben, und wobei das Verfahren weiterhin Folgendes umfasst:

vor dem Rückprojizieren Gewichten der gefilterten Projektionsdaten durch einen inversen Kosinus der Projektionsfächerkoordinate (α).

25. Verfahren nach Anspruch 20, wobei das Rückprojizieren Folgendes umfasst:

Gewichten der gefilterten Projektionsdaten durch einen 1/v-Faktor; und
Rückprojizieren der gefilterten Daten mit der 1/v-Gewichtung.

26. Verfahren nach Anspruch 25, wobei die nativen Scan-Koordinaten eine gekrümmte Geometrie haben, und wobei das Verfahren weiterhin Folgendes umfasst:

vor dem Rückprojizieren Gewichten der gefilterten Projektionsdaten durch einen Kosinus der Projektionsfächerkoordinate (of).

27. Verfahren nach Anspruch 20, wobei die Kernel-Funktion auf einer Hilbert-Transformation basiert.

28. Verfahren nach Anspruch 20, wobei das Berechnen der gefilterten Projektionsdaten weiterhin Folgendes umfasst:

Normalisieren einer Proj ektionslänge von Proj ektionsdaten.

**Revendications**

1. Système d'imagerie par tomographie numérisée à faisceau conique, comprenant :

un émetteur de rayons X (12) qui produit un faisceau conique de rayons X dirigé vers une zone d'examen (14), l'émetteur de rayons X (12) étant disposé de sorte à suivre une trajectoire généralement hélicoïdale autour de la zone d'examen (14) ;

un réseau de détecteurs de rayons X (16) agencé de sorte à détecter le faisceau conique de rayons X après leur passage à travers la zone d'examen (14), le réseau de détecteurs de rayons X (16) générant des données de projection en coordonnées natives de balayage définies en référence au réseau de détecteurs (16) ; et

un processeur de reconstruction exacte (34', 42, 60, 62, 64, 80, 80', 82) qui exécute une reconstruction exacte des données de projection de faisceau conique produites par le réseau de détecteurs (16) en une représentation graphique ;

**caractérisé en ce que** la reconstruction est effectuée dans les coordonnées natives de balayage ;

dans lequel le processeur de reconstruction (34', 42, 60, 62, 64, 80, 80', 82) comprend :

un processeur de dérivation (60) qui calcule une dérivée des données de projection par rapport à un angle hélicoïdal de la trajectoire hélicoïdale à une distance de projection fixe pour générer des données de projection différenciées ;

un processeur de convolution (64) qui effectue une convolution des données de projection différentiées avec une fonction noyau pour produire des données de projection filtrées, la convolution étant effectuée dans les coordonnées natives de balayage ; et

un rétroprojecteur (42, 82) qui rétroprojette les données de projection filtrées pour obtenir une représentation graphique.

2. Système d'imagerie par tomographie numérisée à faisceau conique selon la revendication 1, dans lequel la convolution effectuée par le processeur de convolution (64) est une convolution unidimensionnelle utilisant une fonction noyau basée sur une transformée de Hilbert.

3. Système d'imagerie par tomographie numérisée à faisceau conique selon la revendication 1, dans lequel le processeur de dérivation (60) calcule la dérivée en utilisant un calcul de dérivée discrète par différences finies qui combine arithmétiquement des données de projection avoisinantes sélectionnées.

4. Système d'imagerie par tomographie numérisée à faisceau conique selon la revendication 1, dans lequel le processeur de reconstruction (34', 42, 60, 62, 64, 80, 80', 82) comprend en outre :

un processeur de correction de longueur d'angle de cône (62) qui normalise une longueur de chaque projection en une distance (D) d'émetteur à détecteur.

5. Système d'imagerie par tomographie numérisée à faisceau conique selon la revendication 1, dans lequel le processeur de reconstruction (34', 42, 60, 62, 64, 80, 80', 82) comprend en outre :

un processeur de rééchantillonnage parallèle de données (34') qui rééchantillonne en parallèle les données de projection filtrées, le rétroprojecteur (42) étant un rétroprojecteur parallèle qui rétroprojette les données de rétroprojection filtrées rééchantillonnées en parallèle.

6. Système d'imagerie par tomographie numérisée à faisceau conique selon la revendication 1, dans lequel le réseau de détecteurs ($16_c$) présente une configuration géométrique incurvée des détecteurs, généralement dirigée vers l'émetteur de rayons X, et le système de coordonnées détecteurs est un système incurvé de coordonnées détecteurs ayant des coordonnées natives de balayage comprenant une coordonnée d'angle hélicoïdal ($\lambda$), une coordonnée d'éventail de projection ($\alpha$) et une coordonnée de cône de projection ($\beta$, w).

7. Système d'imagerie par tomographie numérisée à faisceau conique selon la revendication 6, dans lequel le processeur de convolution (64) comprend :

un premier processeur de rééchantillonnage (70) qui rééchantillonne les données de projection différenciées le long de courbes $\kappa$ pour produire des données de projection rééchantillonnées en $\psi$ où $\psi$ représente la courbe $\kappa$ ; et

un processeur de transformation par convolution unidimensionnelle (72) qui effectue une convolution unidimensionnelle des données de projection rééchantillonnées en $\psi$ avec la fonction noyau pour produire des données de projection transformées par convolution, la convolution unidimensionnelle étant à $\psi$ constant dans la coor-

donnée de l'éventail de projection ($\alpha$).

**8.** Système d'imagerie par tomographie numérisée à faisceau conique selon la revendication 7, dans lequel le processeur de transformation par convolution unidimensionnelle (72) effectue la convolution unidimensionnelle telle que :

$$g_{conv}\left(\lambda,\alpha,\Psi\right) = \int_{-\pi/2}^{+\pi/2} d\alpha' h_H\left(\sin\left(\alpha-\alpha'\right)\right) g_{rebin1}\left(\lambda,\alpha',\Psi\right)$$

où $g_{rebin1}(\lambda, \alpha, \psi)$ représente les premières données transformées par convolution, $h_H()$ représente la fonction noyau, et $g_{conv}(\lambda, \alpha, \psi)$ représente les données de projection transformées par convolution.

**9.** Système d'imagerie par tomographie numérisée à faisceau conique selon la revendication 7, dans lequel le processeur de convolution (64) comprend en outre un processeur de pondération (80, 80') qui pondère les données de projection transformées par convolution par un cosinus de la coordonnée d'éventail de projection ($\alpha$) ou un cosinus inverse de la coordonnée d'éventail de projection ($\alpha$) pour produire les données de projection filtrées.

**10.** Système d'imagerie par tomographie numérisée à faisceau conique selon la revendication 9, dans lequel le rétroprojecteur (42, 82) comprend un rétroprojecteur à angle conique (82) qui applique une pondération 1/v aux données de projection filtrées pendant la rétroprojection.

**11.** Système d'imagerie par tomographie numérisée à faisceau conique selon la revendication 6, dans lequel le processeur de reconstruction (34', 42, 60, 62, 64, 80, 80', 82) comprend en outre un processeur de correction de longueur d'angle de cône (62) qui réduit les projections par un cosinus de la coordonnée de cône de projection ($\beta$).

**12.** Système d'imagerie par tomographie numérisée à faisceau conique selon la revendication 1, dans lequel le réseau de détecteurs (16$_f$) a une configuration géométrique des détecteurs sensiblement plane, et le système de coordonnées détecteurs est un système plan de coordonnées détecteurs comprenant des coordonnées natives de balayage ayant une coordonnée d'angle hélicoïdal ($\lambda$), une coordonnée détecteur d'éventail de projection (u), et une coordonnée de cône de projection (w).

**13.** Système d'imagerie par tomographie numérisée à faisceau conique selon la revendication 12, dans lequel le processeur de convolution (64) comprend :

un premier processeur de rééchantillonnage (70) qui rééchantillonne les données de projection différentiées le long de courbes $\kappa$ pour produire des données de projection rééchantillonnées en $\psi$ où $\psi$ représente la courbe $\kappa$ ; et
un processeur de transformation par convolution unidimensionnelle (72) qui effectue une convolution unidimensionnelle des données de projection rééchantillonnées en $\psi$ avec la fonction noyau pour produire des données de projection transformées par convolution, la convolution unidimensionnelle étant à $\psi$ constant dans la coordonnée de l'éventail de projection (u).

**14.** Système d'imagerie par tomographie numérisée à faisceau conique selon la revendication 13, dans lequel le processeur de transformation par convolution unidimensionnelle (72) effectue la convolution unidimensionnelle telle que :

$$g_{conv}\left(\lambda,u,\Psi\right) = \int_{-\infty}^{+\infty} du' h_H\left(\sin\left(u-u'\right)\right) g_{rebin1}\left(\lambda,u',\Psi\right)$$

où $g_{rebin1}(\lambda, u, \psi)$ représente les premières données transformées par convolution, $h_H()$ représente la fonction noyau, et $g_{conv}(\lambda, u, \psi)$ représente les données de projection transformées par convolution.

**15.** Système d'imagerie par tomographie numérisée à faisceau conique selon la revendication 13, dans lequel le processeur de convolution (64) comprend en outre un processeur de rééchantillonnage inversé de la hauteur (74) qui rééchantillonne les données de projection transformées par convolution.

**16.** Système d'imagerie par tomographie numérisée à faisceau conique selon la revendication 1, dans lequel le réseau de détecteurs (16$_c$) présente une configuration géométrique incurvée des détecteurs, généralement dirigée vers l'émetteur de rayons X, et le système de coordonnées détecteurs est un système incurvé de coordonnées détecteurs ayant des coordonnées natives de balayage comprenant une coordonnée d'angle hélicoïdal ($\lambda$), une coordonnée d'éventail de projection ($\alpha$) et une coordonnée de cône de projection ($\beta$, w).

**17.** Système d'imagerie par tomographie numérisée à faisceau conique selon la revendication 1, dans lequel le réseau de détecteurs (16$_f$) présente une configuration géométrique des détecteurs sensiblement plane, et le système de coordonnées détecteurs est un système plan de coordonnées détecteurs comprenant des coordonnées natives de balayage comprenant une coordonnée d'angle hélicoïdal ($\lambda$), une coordonnée détecteur d'éventail de projection (u), et une coordonnée de cône de projection (w).

**18.** Système d'imagerie par tomographie numérisée à faisceau conique selon la revendication 1, dans lequel le processeur de reconstruction (34', 42, 60, 62, 64, 80, 80', 82) comprend :

un processeur de dérivation (60) qui calcule une dérivée finie des données de projection dans les coordonnées natives de balayage le long d'une première direction pour générer des données de projection différenciées ; un processeur de convolution (64) qui effectue une convolution unidimensionnelle des données de projection différenciées dans les coordonnées natives de balayage le long d'une deuxième direction qui est différente de la première direction, afin de produire des données de projection filtrées ; et un rétroprojecteur (42, 82) qui rétroprojette les données de projection filtrées pour obtenir une représentation graphique.

**19.** Système d'imagerie par tomographie numérisée à faisceau conique selon la revendication 1, dans lequel les coordonnées natives de balayage comprennent au moins un angle hélicoïdal ($\lambda$), une coordonnée d'éventail de projection ($\alpha$, u) et une coordonnée de cône de projection ($\beta$, w).

**20.** Procédé de reconstruction exacte pour reconstruire des données de projection tomographique numérisées à faisceau conique comprenant des coordonnées natives de balayage qui comprennent au moins un angle hélicoïdal ($\lambda$), une coordonnée d'éventail de projection ($\alpha$, u) et une coordonnée de cône de projection ($\beta$, w), **caractérisé en ce que** le procédé est exécuté dans les coordonnées natives de balayage ; ledit procédé comprenant les étapes consistant à :

calculer des données de projection filtrées en combinant : le calcul d'une dérivée des données de projection par rapport à l'angle hélicoïdal ($\lambda$) pour une direction de projection fixe, et la convolution des données de projection avec une fonction noyau, la convolution étant effectuée dans les coordonnées natives de balayage ; et rétroprojeter les données de projection filtrées pour obtenir une représentation graphique.

**21.** Procédé selon la revendication 20, dans lequel le calcul d'une dérivée comprend le calcul de chaque donnée de projection différentiée par une dérivée par différences finies sur la base d'une pluralité de données de projection qui avoisinent ladite donnée de projection différenciée.

**22.** Procédé selon la revendication 20, dans lequel la convolution comprend les étapes consistant à :

rééchantillonner les données de projection par rapport à des plans $\kappa$ (K($\lambda$, $\psi$) pour grouper les projections des données de projection par coordonnée $\psi$ de plan $\kappa$ ; et effectuer une convolution unidimensionnelle des données de projection rééchantillonnées avec la fonction noyau, la convolution unidimensionnelle étant effectuée par rapport à la coordonnée de l'éventail de projection ($\alpha$, u), la convolution unidimensionnelle étant à coordonnée $\psi$ fixe sur le plan $\kappa$.

**23.** Procédé selon la revendication 20, dans lequel la rétroprojection comprend les étapes consistant à :

rééchantillonner en parallèle les données de projection filtrées pour obtenir des vues de projection parallèles ; et rétroprojeter les vues de projection parallèles.

**24.** Procédé selon la revendication 23, dans lequel les coordonnées natives de balayage ont une configuration géométrique incurvée, le procédé comprenant en outre, avant la rétroprojection, l'étape consistant à pondérer les données de projection filtrées par un cosinus inverse de la coordonnée d'éventail de projection ($\alpha$).

**25.** Procédé selon la revendication 20, dans lequel la rétroprojection comprend les étapes consistant à :

> pondérer les données de projection filtrées par un facteur 1/v ; et
> rétroprojeter les données de projection filtrées avec la pondération 1/v.

**26.** Procédé selon la revendication 25, dans lequel les coordonnées natives de balayage présentent une configuration géométrique incurvée, le procédé comprenant en outre, avant la rétroprojection, l'étape consistant à pondérer les données de projection filtrées par un cosinus de la coordonnée d'éventail de projection ($\alpha$).

**27.** Procédé selon la revendication 20, dans lequel la fonction noyau est basée sur une transformée de Hilbert.

**28.** Procédé selon la revendication 20, dans lequel le calcul de données de projection filtrées comprend en outre l'étape consistant à normaliser une longueur de projection des données de projection.

FIG 1

EP 1 599 836 B1

FIG 2

FIG 3

FIG 4

EP 1 599 836 B1

FIG 5

FIG 6

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6104775 A, Tuy

- US 27481602 A, Heuscher

**Non-patent literature cited in the description**

- **Katsevich et al.** *Proceedings SPIE Medical Imaging Conference,* February 2003, 663-674